# EUROPEAN PATENT APPLICATION

(11) **EP 4 169 516 A1**
(43) Date of publication of application: **26.04.2023**
(21) Application number: 22185508.3
(22) Date of filing: 18.07.2022
(51) Int. Cl.: A61K 31/55, A61K 9/20

(54) **PREVENTION OF THE CONVERSION OF PHARMACEUTICAL AGENTS INTO TOXIC N-NITROSAMINE COMPOUNDS**

(30) Priority: 05.11.2021 IN 202111050763; 15.07.2022 US 202217866382
(71) Applicant: Alfred E. Tiefenbacher (GmbH & Co. KG), 22767 Hamburg (DE)
(72) Inventor: Döbber, Alexander, 22767 Hamburg (DE); Sagili, Manohar Reddy, 502319 Hyderabad (IN); Gujjar, Chaitanya Yogananda, 502319 Hyderabad (IN); Kalamata, Venkatasimhadri Naidu, 22767 Hamburg (DE); Fitzner, Ansgar, 22767 Hamburg (DE); Mankala, Santhosh Kumar, 502319 Hyderabad (IN); Rallabandi, Bala Ramesha Chary, 502319 Hyderabad (IN); Madallapalli, Kiran Kumar, 502319 Hyderabad (IN)
(74) Representative: Hamm&Wittkopp Patentanwälte PartmbB

(57) **Abstract**

In the solid pharmaceutical compositions of the present invention, the formation of toxic N-nitroso-derivatives of the contained agents comprising a dialkylamino- or a trialkylamino-group is prevented providing long-term storage stable formulations of inherently problematic drugs, such as varenicline (e.g., in form of its tartrate or the citrate). Means for achieving the long-term storage stable formulations of the present invention are formulating the crucial active ingredients with antioxidants, using specific packaging materials for the pharmaceutical compositions of the present invention, and/or employing specific manufacturing processes.

## Description

The present invention relates to the prevention of the formation of toxic N-nitroso-derivatives of pharmaceutical agents within pharmaceutical dosage forms upon reaction of a secondary or tertiary amine with a nitrosating agent such as nitrites within a final dosage form. It is well known that compounds that comprise a secondary dialkylamino- or a tertiary trialkylamino-group are at risk to react into N-nitrosamine-derivatives that are highly toxic and, e.g., carcinogenic.

In the case of such crucial pharmaceutical agents, the source for the nitrite anions that react with the compound's amino function may be part of the agent's synthesis or come with the excipients that are used in the formulation of the final dosage forms. For example, excipients that stem from natural materials, such as MCC, starch, and starch derivatives, ubiquitously comprise some nitrite salts, which can react with the amino function of the pharmaceutical agent to form problematic N-nitrosamine-derivatives.

It is the main object of the present invention to provide a solution to the problematic formation of N-nitroso-derivatives of pharmaceutical agents within the solid pharmaceutical dosage forms of crucial pharmaceutical agents (comprising one or more secondary or tertiary amino function that is prone to convert into or to release toxic, in particular carcinogenic, N-nitroso-derivatives), and to provide storage stable pharmaceutical compositions.

It is a further object of the present invention to provide storage stable pharmaceutical compositions of varenicline or of pharmaceutically acceptable salts thereof, whereby the formation of toxic, in particular carcinogenic, N-nitroso-derivatives is prevented or, at least, reduced to acceptable levels.

### Background of the invention

There are several pharmaceutical products known to be at risk for potential N-nitrosamine contamination. In most cases the risk for formation of N-nitrosamines such as NDMA and NDEA arises from the manufacturing of the drug substance and is introduced by latter into the drug product (e.g. Valsartan or Metformin case). The current invention relates to the special case of formation of a N-nitroso-derivative of the drug substances within the final drug product either during manufacturing or storage of latter.

It has been proposed that during storage of the corresponding final dosage forms of secondary or tertiary amines, the amino-groups are converted into highly toxic N-nitrosamines. Only in cases, wherein at least one of the alkyl-substituents comprises a tertiary carbon atom in alpha-position, the resulting N-nitrosamine may not be carcinogenic.

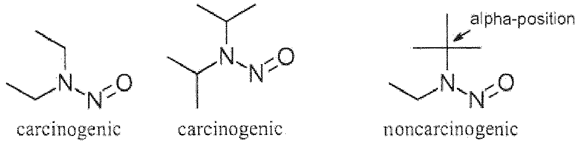

The pharmaceutical agent varenicline, 7,8,9,10-tetrahydro-6,10-methano-6H-pyracino[2,3-h]-[3]benzacepine, represents a high-affinity partial agonist of the α4β2 nicotinic acetyl colin receptor subtype (nACh) that leads to the release of the neurotransmitter dopamine in the *nucleus accumbens* reward center of the brain when activated, and therefore, varenicline has the capacity to reduce the feelings of craving caused by smoking cessation. Thus, varenicline and its pharmaceutical compositions are used to treat tobacco use disorder.

As observed by several medical authorities, during storage of art-known pharmaceutical compositions comprising varenicline, e.g. in form of its tartrates or citrates, the secondary dialkylamino-function tends to react with traces of nitrite anions to form possibly carcinogenic N-nitroso-varenicline.

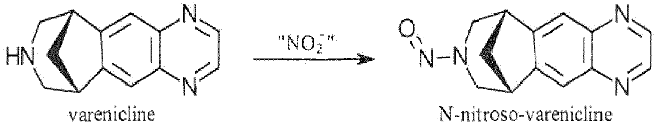

Thus, there is an urgent need for providing new pharmaceutical compositions of the crucial pharmaceutical agents, which comprise a secondary or tertiary amino function that are at risk to react into toxic and/or carcinogenic N-nitrosamine-derivatives or to release such toxic and/or carcinogenic N-nitrosamine-derivatives, that are long-term stable. In particular, there is a need to provide a storage stable composition of varenicline and its pharmaceutically acceptable salts that do not comprise unacceptable amounts of N-nitroso-varenicline even after long periods of storage.

### Detailed description

Based on the standards established by the health authorities, the maximum allowed daily intake of N-nitroso-varenicline is set to 37 ng/day which results in a limit of below 18.5 ppm over the entire shelf life of the pharmaceutical product (relative to the weight of varenicline free base in the pharmaceutical formulation) and calculated on the maximum daily intake of 2 mg varenicline free base.

In the case of varenicline's free base, varenicline citrate, and varenicline tartrate it was observed that the compositions according to the prior art (not comprising any antioxidant) contain unacceptable amounts of N-nitroso-varenicline already after a short storage period, such as after six months (at 25 °C / 60 %rH) in the primary packaging; similar conversions into the problematic N-nitroso-compound have been observed under stress conditions already after shorter periods of time, e.g. after a period of 2 weeks at 60 °C under open exposure (at any humidity between 30 and 75 %rH) or after a period of 6 months at 40 °C / 75 %rH in the primary packaging. In each of these cases, the concentration of the undesired N-nitroso-varenicline increased over time and reached crucial levels far before the composition's official shelf-life. In the present application, the terms "N-nitroso-varenicline", "IM2 (impurity)" and "Varenicline nitroso impurity" are used synonymously. In the present application, "rH" or "RH" means "relative humidity".

By contrast, when pharmaceutical agents comprising a dialkylamino- or a trialkylamino-group (such as varenicline's free base, varenicline citrate, and varenicline tartrate) are formulated in accordance with the present invention (including an antioxidant), the formation of toxic and carcinogenic N-nitroso-compounds can be prevented or, at least, slowed to such a degree that their concentration remains within the acceptable range for the formulation's entire shelf-life. This highly desired behavior can be documented by tests under stress conditions for a period of 2 weeks at 60 °C under open exposure (at any humidity between 30 and 75 %rH), or over six months (at 25 °C / 60 %rH) in the primary packaging, or for a period of six months at 40 °C / 75 %rH in the primary packaging.

It has been found in the extensive experiments leading to the present invention that said undesired conversion into N-nitrosamine-derivates during both manufacturing of the pharmaceutical dosage form as well as over its shelf-life can be prevented or, at least, slowed down by formulating the pharmaceutical agent together with at least one pharmaceutically acceptable antioxidant into a solid dosage form, such as a tablet, a capsule, pellets, or granules, preferably a tablet. According to the present invention, an antioxidant typically is any chemical compound that prevents or delays the oxidation of pharmaceutical active ingredients.

Correspondingly, the present invention refers to a pharmaceutical composition, which is an oral solid dosage form, containing a pharmaceutical agent comprising a dialkylamino- or a trialkylamino-group and one or more pharmaceutically acceptable excipient(s),
wherein over a period of
- 2 weeks at 60 °C under open exposure (at any humidity between 30 and 75 %rH), or
- 6 months at 40 °C / 75 %rH in a primary packaging, or
- 6 months at 25 °C / 60 %rH in a primary packaging,
the concentration of the corresponding N-nitroso-derivative of the pharmaceutical agent remains below 50 ppm, preferably below 30 ppm, more preferably below 18.5 ppm, even more preferably below 10 ppm, even more preferably below 5 ppm and most preferably below 2 ppm, such as below 1 ppm (relative to the weight of the free base of the pharmaceutical agent in the pharmaceutical composition).

In the present application, the primary packaging can be any conventional primary packaging known in the art.

Also, in the present application, the concentration of an N-nitroso-derivative of a pharmaceutical agent relates to the weight of the free base of said pharmaceutical agent, if not stated otherwise.

The present invention also refers to a combination of such a pharmaceutical composition with a primary packaging, wherein said pharmaceutical composition is packed with said primary packaging, and wherein over a period of
- 2 weeks at 60 °C under open exposure, i.e. exposure of the unpacked pharmaceutical composition as such, at any humidity between 30 and 75 %rH, or
- 6 months at 40 °C / 75 %rH in the primary packaging, or
- 6 months at 25 °C / 60 %rH in the primary packaging,
the concentration of the corresponding N-nitroso-derivative of the pharmaceutical agent remains below 50 ppm, preferably below 30 ppm, more preferably below 18.5 ppm, even more preferably below 10 ppm, even more preferably below 5 ppm and most preferably below 2 ppm, such as below 1 ppm (relative to the weight of the free base of the pharmaceutical agent in the pharmaceutical composition).

In a preferred embodiment of the pharmaceutical composition, the concentration of the corresponding N-nitroso-derivative of the pharmaceutical agent remains below 50 ppm, preferably below 30 ppm, more preferably below 18.5 ppm, even more preferably below 10 ppm, even more preferably below 5 ppm and most preferably below 2 ppm, such as below 1 ppm (relative to the weight of the free base of the pharmaceutical agent in the pharmaceutical composition) over a period of 2 weeks at 60 °C under open exposure (at any humidity between 30 and 75 %rH, such as e.g. 40 %rH).

In another preferred embodiment of the pharmaceutical composition, the concentration of the corresponding N-nitroso-derivative of the pharmaceutical agent remains below 50 ppm, preferably below 30 ppm, more preferably below 18.5 ppm, even more preferably below 10 ppm, even more preferably below 5 ppm and most preferably below 2 ppm, such as below 1 ppm (relative to the weight of the free base of the pharmaceutical agent in the pharmaceutical composition) over a period of 6 months at 40 °C / 75 %rH in a primary packaging and the primary packaging, respectively.

In still another preferred embodiment of the pharmaceutical composition, the concentration of the corresponding N-nitroso-derivative of the pharmaceutical agent remains below 50 ppm, preferably below 30 ppm, more preferably below 18.5 ppm, even more preferably below 10 ppm, even more preferably below 5 ppm and most preferably below 2 ppm, such as below 1 ppm (relative to the weight of the free base of the pharmaceutical agent in the pharmaceutical composition) over a period of 6 months at 25 °C / 60 %rH in a primary packaging and the primary packaging, respectively.

In a particularly preferred embodiment of the pharmaceutical composition, the concentration of the corresponding N- nitroso-derivative of the pharmaceutical agent remains below 50 ppm, preferably below 30 ppm, more preferably below 18.5 ppm, even more preferably below 10 ppm, even more preferably below 5 ppm and most preferably below 2 ppm, such as below 1 ppm (relative to the weight of the free base of the pharmaceutical agent in the pharmaceutical composition) over a period of
- 2 weeks at 60 °C under open exposure (at any humidity between 30 and 75 %rH), and
- 6 months at 40 °C / 75 %rH in a primary packaging and the primary packaging, respectively, and
- 6 months at 25 °C / 60 %rH in a primary packaging and the primary packaging, respectively.

In the present invention, the pharmaceutical agent preferably is varenicline or a pharmaceutically acceptable salt thereof, preferably a pharmaceutically acceptable salt thereof. Said pharmaceutically acceptable salt of varenicline preferably is the tartrate salt or the citrate salt.

Possible antioxidants to be used in accordance with the present invention are alpha-tocopherol, ascorbic palmitate, butylated hydroxyanisol, butylated hydroxytoluene, erythorbic acid, Vitamin E polyethylene glycol succinate, methionine, potassium metabisulfite, sodium metabisulfite, sodium ascorbate, sodium bisulfite, sodium formaldehyde sulfoxylate, or sodium sulfite, sodium thiosulfate, ascorbic acid or a salt thereof, or propyl gallate. Preferably, the antioxidant is propyl gallate or ascorbic acid or a salt thereof. Most preferably the antioxidant is propyl gallate. In another embodiment, the antioxidant is ascorbic acid or a salt thereof. To achieve the desired stability, the pharmaceutical composition comprises the antioxidant(s) in a concentration of typically at least 0.1 w.-%, preferably at least 0.25 w.-% (relative to the weight of the entire pharmaceutical composition); more preferably in a concentration of 0.5-20 w.-%, such as 1-15 w.-% (relative to the weight of the entire pharmaceutical composition). If the antioxidant used is ascorbic acid or a salt thereof, it is typically used at a concentration of at least 2 w.-% and preferably of 5-15 w.-% (relative to the weight of the entire pharmaceutical composition). Most preferably, the antioxidant is propyl gallate and its concentration typically is 0.25-1.75 w.-%, preferably 0.5-1.5 w.-%, and most preferably 0.75-1.25 w.-% (relative to the weight of the entire pharmaceutical composition). In this application, "w.-%" and "wt.-%" are used synonymously and mean "weight percent".

In a preferred embodiment of the process of manufacturing the inventive pharmaceutical composition being an oral solid dosage form, pharmaceutically acceptable excipient(s) such as a diluent and/or a binder, preferably a diluent and a binder, are wet granulated with an aqueous dispersion of an antioxidant so that wet granules are formed, wherein after drying and optionally milling said wet granules, the resulting dried granules are blended with the pharmaceutical agent and optionally another pharmaceutically acceptable excipient such as a lubricant and/or an organic acid, preferably a lubricant and an organic acid.

In said wet granulation step, the aqueous dispersion preferably is an aqueous solution of an antioxidant such as e.g. propyl gallate. Surprisingly, it has found that a treatment by wet granulation according to the present invention enables that any undesired conversion into N-nitrosamine-derivates during both manufacturing of the pharmaceutical dosage form as well as over its shelf-life can be prevented or, at least, slowed down in a particularly effective manner. In the present application, an aqueous solution is understood as a preferred embodiment of an aqueous dispersion. In a preferred embodiment, the aqueous dispersion also comprises a pharmaceutically acceptable alcohol such as isoproponaol or ethanol, preferably ethanol. In another preferred embodiment, the aqueous dispersion also comprises a pharmaceutically acceptable organic acid such as citric acid or tartaric acid. The organic acid acts as a synergist. If the pharmaceutically acceptable salt of varenicline is the tartrate, then the organic acid preferably is tartaric acid. If the pharmaceutically acceptable salt of varenicline is the citrate, then the organic acid preferably is citric acid. In the most preferred embodiment, the aqueous dispersion both comprises a pharmaceutically acceptable alcohol as well as an organic acid.

Preferably, the blend of granules, pharmaceutical agent and optionally another pharmaceutically acceptable excipient is dry granulated afterwards, e.g. by roller compaction. According to the present application, in the dry granulation step, the blend is typically milled to granules after its compaction. The resulting granules can then e.g. be compressed to a tablet, typically together with a non-granular pharmaceutically acceptable excipient such as a lubricant, or filled into capsules. Preferably, they are compressed to a tablet. In an alternative embodiment, the blend can also be directly compressed to a tablet. However, dry granulation typically has several advantages compared to direct compression such as better flowability and higher content uniformity of the pharmaceutical agent in the resulting final tablet.

Correspondingly, the pharmaceutical composition of the present invention is preferably obtained by wet granulating any pharmaceutically acceptable excipient(s) such as a diluent and/or a binder, preferably a diluent and a binder, with an aqueous dispersion as defined above so that wet granules are formed, wherein after drying and optionally milling said wet granules, the resulting dried granules are blended with the pharmaceutical agent and optionally another pharmaceutically acceptable excipient such as a lubricant and/or an organic acid, preferably a lubricant and an organic acid, preferably followed by dry granulating the resulting blend. In a preferred embodiment, a tablet is obtained afterwards by compressing the resulting granules, typically together with a non-granular pharmaceutically acceptable excipient such as a lubricant, or capsules are obtained by filling the corresponding capsule shells with said granules. Alternatively, a tablet can be obtained by direct compression of the afore-mentioned blend.

In an alternative embodiment of the production of the inventive pharmaceutical composition, pharmaceutically acceptable excipient(s) such as a diluent and/or a binder, preferably a diluent and a binder, are not wet granulated with an aqueous dispersion of an antioxidant, but are directly blended with the pharmaceutical agent, the antioxidant and optionally another pharmaceutically acceptable excipient such as a lubricant and/or an organic acid, preferably a lubricant and an organic acid. Preferably, the resulting blend is dry granulated afterwards, e.g. by roller compaction. The resulting granules can then e.g. be compressed to a tablet, typically together with a non-granular pharmaceutically acceptable excipient such as a lubricant, or filled into capsules. Preferably, they are compressed to a tablet. In an alternative embodiment, the blend can also be directly compressed to a tablet.

Correspondingly, the pharmaceutical composition of the present invention can alternatively be obtained by directly blending the pharmaceutical agent with pharmaceutically acceptable excipient(s) such as a diluent, a binder, an organic acid and/or a lubricant, and typically subsequently dry granulating the resulting blend. Preferably, a tablet is obtained afterwards by compressing the resulting granules, typically together with a non-granular excipient such as a lubricant, or capsules are obtained by filling the corresponding capsule shells with said granules. Alternatively, a tablet can be obtained by direct compression of the afore-mentioned blend.

The inventive pharmaceutical composition may be packed with any conventional primary packaging known in the art, for example blisters, or polypropylene or polyethylene (e.g. HDPE, high density polyethylene) bottles. Preferably, the inventive pharmaceutical composition, such as tablet composition, is packed with a HDPE bottle, optionally comprising a desiccant such as silica gel (e.g. in an amount of 2 g). In another preferred embodiment, the pharmaceutical composition, such as tablet composition, is packed with a blister known in the art, e.g., PVC-Alu, PVC/PVDC-Alu, PVC/PCTFE-Alu, PVC/PCTFE/PVC-Alu, PVC/PE/PVDC-Alu, and Aluminum-Aluminum (Alu-Alu) blister, more preferably with PVC-Alu blister.

Preferably, the solid oral dosage form is a tablet. The tablet may be obtained by direct compression. Preferably, it is obtained by granulation compression exhibiting a granular composition (forming the intragranular composition in the tablet core) and a non-granular composition (forming the extragranular composition in the tablet core). The granular composition used is typically produced by dry granulation, e.g. using roller compaction. Dry granulation typically has several advantages compared to direct compression such as better flowability and better content uniformity of the pharmaceutical agent in the resulting final product.

In the present application, if the oral dosage form is a tablet, the concentration of any antioxidant refers to the tablet core, i.e. any tablet coating is not regarded as part of the pharmaceutical composition in this context (analogously, if the oral dosage form is a capsule, the capsule shell is not regarded as part of the pharmaceutical composition in this context). In such a tablet, the pharmaceutical agent and the antioxidant typically are present in the same composition. This means that i) in case the inventive pharmaceutical tablet is obtained by direct compression, both the pharmaceutical agent and the antioxidant are typically present in the mixture of ingredients to be compressed to tablet cores, and ii) in case the inventive pharmaceutical tablet composition is obtained by granulation compression exhibiting a granular composition (forming the intragranular composition in the tablet core) and a non-granular composition (forming the extragranular composition in the tablet core), both the pharmaceutical agent and the antioxidant are typically present either in the intragranular composition or in the extragranular composition or both the pharmaceutical agent and the antioxidant are present in both the intragranular and the extragranular composition respectively. Notwithstanding, the antioxidant may additionally be present in the intragranular or extragranular composition, which does not comprise any pharmaceutical agent. In case the inventive pharmaceutical tablet composition is obtained by such a granulation compression, preferably both, the pharmaceutical agent and the antioxidant, are present in the intragranular composition.

The present pharmaceutical formulation (composition), such as e.g. tablet, typically comprises a diluent, a binder, and a lubricant. In a preferred embodiment, it comprises microcrystalline cellulose as diluent. In another preferred embodiment, it comprises pregelatinized starch as binder. In still another preferred embodiment, it comprises magnesium stearate as lubricant. In a particularly preferred embodiment, it comprises all of said ingredients, i.e. microcrystalline cellulose, pregelatinized starch and magnesium stearate.

Further preferred embodiments of the pharmaceutical composition according to the present invention and its process of manufacture are described in the dependent claims. In the present application, preferred embodiments of the pharmaceutical composition are also preferred embodiments of its process of manufacture and vice versa.

### Experimental section:

Film-coated tablets comprising varenicline citrate or varenicline tartrate (in dosages corresponding to 0.5 mg or 1 mg free varenicline base) were prepared according to the compositions and the general procedures below (in this application, "%" means "weight-%" if not stated otherwise):

### General manufacturing procedure for comparative compositions C1 and C2, and for inventive compositions E1 - E14:

1. Blend Varenicline Citrate^{∗} (if any) and excipients of Stage-A.
2. Perform the compaction of the step 1 blend using suitable equipment and process parameters.
3. Mill the step 2 compacted mass using suitable equipment with 1.0 mm screen.
4. Lubricate the step 3 blend with the magnesium stearate of stage-B.
5. Compress the tablets.
6. Prepare the coating suspension using coating material and purified water in the stage-C.
7. Using step-6 coating suspension, perform the coating on step-5 compressed tablets using suitable coating equipment and process parameters to reach the target build up.
^{∗} content of N-nitroso-varenicline in used API batches is below the level of detection (LOD) of the LCMS analytical used method being 0.2 ppm (calculated w.r. to Varenicline free base).

### General manufacturing procedure for inventive compositions E15 - E21 (using additional wet granulation step):

1. Dry mixing the excipients of Stage-A.
2. Wet granulate the step 1 blend with the binder solution of Stage-B using suitable equipment and process parameters.
3. Dry the wet granules at a controlled temperature not exceeding 60°C to a consistent weight.
4. Mill the step 3 dried granules using suitable equipment with screen size of 18 R (457µ) followed by 11R (279µ).
5. Blend the varenicline citrate^{∗} or tartrate^{∗}, the citric acid or tartaric acid (if any) and the magnesium stearate of Stage-C with the step 4 milled granules.
6. Perform the compaction of the step 5 blend using suitable equipment and process parameters.
7. Mill the step 6 compacted mass using suitable equipment with 1.0 mm screen.
8. Lubricate the step 7 blend with the magnesium stearate of stage-D.
9. Compress the tablets.
10. Prepare the coating suspension using coating material and purified water in the stage-E.
11. Using step-10 coating suspension, perform the coating on step-9 compressed tablets using suitable coating equipment and process parameters to reach the target build up.
^{∗} content of N-nitroso-varenicline in used API batches is below the level of detection (LOD) of the LCMS analytical method used being 0.2 ppm (calculated w.r. to Varenicline free base).

**Compositions** (in Examples E15 - E21, ethanol and water will be removed during general manufacturing procedure):

**Table 1: Comparative compositions.**

| **Example Number** | **C1** | **C2** |
|---|---|---|
| **Strength** | **1 mg strength** | **0.5 mg strength** |
| **Antoxidant concentration** | **0 %** | **0 %** |
| **Ingredients** | **mg/tablet** | **mg/tablet** |

| **Stage** - **A (Blending & Compaction)** | | |
|---|---|---|
| Varenicline Citrate | 1.909 | 0.955 |
| MCC | 117.891 | 58.945 |
| Starch Pregelatinized | 80.00 | 40.000 |
| Magnesium Stearate | 0.100 | 0.050 |

| **Stage** - **B (Lubrication & Compression)** | | |
|---|---|---|
| Magnesium Stearate | 0.100 | 0.050 |
| **Core tablet weight (mg)** | **200.000** | **100.000** |

| **Stage** - **C (Coating)** | | |
|---|---|---|
| Opadry^{®} Blue | 10.000 | 5.000 |
| Water purified | q.s. | q.s. |
| **Coated tablet weight (mg)** | **210.000** | **105.000** |

**Table 2: Trials according to the invention with antioxidant ascorbic acid.**

| **Example Number** | **E1** | **E2** | **E3** | **E4** | **E5** | **E6** | **E7** |
|---|---|---|---|---|---|---|---|
| **Antoxidant concentration** | **0.1%** | **0.5%** | **1%** | **2%** | **5%** | **10%** | **15%** |
| **Ingredients (mg/ tablet)** | | | | | | | |
| **Stage** - **A (Blending & Compaction)** | | | | | | | |
| Varenicline Citrate | 1.909 | 1.909 | 1.909 | 1.909 | 1.909 | 1.909 | 1.909 |
| Ascorbic acid | 0.200 | 1.000 | 2.000 | 4.000 | 10.000 | 20.000 | 30.000 |
| MCC | 117.691 | 116.891 | 115.891 | 113.691 | 107.891 | 97.891 | 87.891 |
| Starch Pregelatinized | 80.000 | 80.000 | 80.000 | 80.000 | 80.000 | 80.000 | 80.000 |
| Magnesium Stearate | 0.100 | 0.100 | 0.100 | 0.100 | 0.100 | 0.100 | 0.100 |

| **Stage** - **B (Lubrication & Compression)** | | | | | | | |
|---|---|---|---|---|---|---|---|
| Magnesium Stearate | 0.100 | 0.100 | 0.100 | 0.100 | 0.100 | 0.100 | 0.100 |
| **Core tablet weight (mg)** | **200.000** | **200.000** | **200.000** | **200.000** | **200.000** | **200.000** | **200.000** |

| **Stage** - C **(Coating)** | | | | | | | |
|---|---|---|---|---|---|---|---|
| Opadry^{®} Blue | 10.000 | 10.000 | 10.000 | 10.000 | 10.000 | 10.000 | 10.000 |
| Water purified | q.s | q.s | q.s | q.s | q.s | q.s | q.s |
| **Coated tablet weight (mg)** | **210.000** | **210.000** | **210.000** | **210.000** | **210.000** | **210.000** | **210.000** |

**Table 3: Trials with antioxidant propyl gallate (according to the invention).**

| **Example Number** | **E8** | **E9** | **E10** | **E11** | **E12** |
|---|---|---|---|---|---|
| **Antioxidant concentration** | **1.0 %** | **0.25%** | **0.50%** | **0.75%** | **1.75%** |
| Ingredients **(mg/tablet)** | | | | | |
| **Stage** - **A (Blending & Compaction)** | | | | | |
| Varenicline Citrate | 1.909 | 1.909 | 1.909 | 1.909 | 1.909 |
| Propyl gallate | 2.000 | 0.500 | 1.000 | 1.500 | 3.500 |
| Microcrystalline Cellulose | 115.891 | 117.391 | 116.891 | 116.391 | 114.391 |
| Starch Pregelatinized | 80.000 | 80.000 | 80.000 | 80.000 | 80.000 |
| Magnesium Stearate | 0.100 | 0.100 | 0.100 | 0.100 | 0.100 |

| **Stage** - **B (Lubrication & Compression)** | | | | | |
|---|---|---|---|---|---|
| Magnesium Stearate | 0.100 | 0.100 | 0.100 | 0.100 | 0.100 |
| **Core tablet weight (mg)** | **200.000** | **200.000** | **200.000** | **200.000** | **200.000** |

| **Stage** - **C (Coating)** | | | | | |
|---|---|---|---|---|---|
| Opadry^{®} Blue | 10.000 | 10.000 | 10.000 | 10.000 | 10.000 |
| Water purified | q.s. | q.s. | q.s. | q.s. | q.s. |
| **Coated tablet weight** (mg) | **210.000** | **210.000** | **210.000** | **210.000** | **210.000** |

**Table 4: Trial with antioxidant Sodium Ascorbate (according to the invention).**

| **Example Number** | **E13** |
|---|---|
| **Antioxidant concentration** | **5.0 %** |
| **Ingredients (mg/tablet)** | |
| **Stage** - **A (Blending & Compaction)** | |
| Varenicline Citrate | 1.909 |
| Sod. Ascorbate | 10.000 |
| Microcrystalline Cellulose | 107.891 |
| Starch Pregelatinized | 80.000 |
| Magnesium Stearate | 0.100 |

| **Stage** - **B (Lubrication & Compression)** | |
|---|---|
| Magnesium Stearate | 0.100 |
| **Core tablet weight (mg)** | **200.000** |

| **Stage** - **C (Coating)** | |
|---|---|
| Opadry^{®} Blue | 10.000 |
| Water purified | q.s. |
| **Coated tablet weight (mg)** | **210.000** |

**Table 5: Trial with antioxidant Sodium metabisulfite (according to the invention).**

| **Example Number** | **E14** |
|---|---|
| **Antioxidant concentration** | **5.0 %** |
| **Ingredients (mg/tablet)** | |
| **Stage - A (Blending & Compaction)** | |
| Varenicline Citrate | 1.909 |
| Sod. Metabisulfite | 10.000 |
| Microcrystalline Cellulose | 107.891 |
| Starch Pregel. | 80.000 |
| Magnesium Stearate | 0.100 |

| **Stage - B (Lubrication & Compression)** | |
|---|---|
| Magnesium Stearate | 0.100 |
| **Core tablet weight (mg)** | **200.000** |

| **Stage - C (Coating)** | |
|---|---|
| Opadry^{®} Blue | 10.000 |
| Water purified | q.s. |
| **Coated tablet weight (mg)** | **210.000** |

**Table 6: Trials with varenicline citrate and antioxidant propyl gallate (according to the invention, using wet granulation).**

| **Example Number** | **E15** | **E16** | **E17** | **E18** | **E19** |
|---|---|---|---|---|---|
| **Antioxidant concentration** | **1.0 %** | **1.0%** | **1.0 %** | **1.0 %** | **1.0 %** |
| **Ingredients (mg/tablet)** | | | | | |
| **Stage** - **A (Dry Mixing)** | | | | | |
| Microcrystalline Cellulose | 115.891 | 115.891 | 113.982 | 112.071 | 56.036 |
| Starch Pregel. | 80.000 | 80.000 | 80.000 | 80.000 | 40.000 |

| **Stage** - **B (Binder Solution)** | | | | | |
|---|---|---|---|---|---|
| Propyl gallate | 2.000 | 2.000 | 2.000 | 2.000 | 1.000 |
| Citric acid | - | - | 1.909 | 1.910 | 0.955 |
| Ethanol | - | q.s to make 25% w/w of dry mix using aqueous solution comprising ethanol | q.s to make 25% w/w of dry mix using aqueous solution comprising ethanol | 4.000 | 2.000 |
| Water purified | q.s. to make 25% w/w of dry mix | - | - | 54.414 | 27.212 |

| **Stage** - **C (Blending and Compaction)** | | | | | |
|---|---|---|---|---|---|
| Varenicline citrate | 1.909 | 1.909 | 1.909 | 1.909 | 0.955 |
| Citric acid anhydrous | - | - | - | 1.910 | 0.955 |
| Magnesium stearate | 0.100 | 0.100 | 0.100 | 0.100 | 0.050 |

| **Stage - D (Lubrication and Compression)** | | | | | |
|---|---|---|---|---|---|
| Magnesium stearate | 0.100 | 0.100 | 0.100 | 0.100 | 0.050 |
| **Core tablet weight (mg)** | **200.000** | **200.000** | **200.000** | **200.000** | **100.000** |

| **Stage** - **E (Coating)** | | | | | |
|---|---|---|---|---|---|
| Opadry^{®} Blue | 10.000 | 10.000 | 10.000 | 10.000 | 5.000 |
| Water purified | q.s. | q.s. | q.s. | q.s. | q.s. |
| **Coated tablet weight (mg)** | **210.000** | **210.000** | **210.000** | **210.000** | **105.000** |

**Table 7: Trials with varenicline tartrate and antioxidant propyl gallate (according to the invention, using wet granulation).**

| **Example Number** | **E20** | **E21** |
|---|---|---|
| **Antioxidant concentration** | **1.0 %** | **1.0 %** |
| **Ingredients (mg/tablet)** | | |
| **Stage** - **A (Dry Mixing)** | | |
| Microcrystalline Cellulose | 112.670 | 112.670 |
| Starch Pregel. | 80.000 | 80.000 |

| **Stage** - **B (Binder Solution)** | | |
|---|---|---|
| Propyl gallate | 2.000 | 2.000 |
| Organic acid | 1.710 (tartaric acid) | 1.710 (citric acid anhydrous) |
| Ethanol | 4.000 | 4.000 |
| Water purified | 54.414 | 54.414 |

| **Stage** - **C (Blending and Compaction)** | | |
|---|---|---|
| Varenicline tartrate | 1.710 | 1.710 |
| Organic acid | 1.710 (tartaric acid) | 1.717 (citric acid anhydrous) |
| Magnesium stearate | 0.100 | 0.100 |

| **Stage** - **D (Lubrication and Compression)** | | |
|---|---|---|
| Magnesium stearate | 0.100 | 0.100 |
| **Core tablet weight (mg)** | **200.000** | **200.000** |

| **Stage** - **E (Coating)** | | |
|---|---|---|
| Opadry^{®} Blue | 10.000 | 10.000 |
| Water purified | q.s. | q.s. |
| **Coated tablet weight (mg)** | **210.000** | **210.000** |

### Analysis of compositions:

### Test conditions:

The chemical stability of the comparative and inventive pharmaceutical compositions (tablets) is tested in a conventional manner, in particular after 6 months storage under long term conditions (25°C/60 % rH in primary packaging intended for the commercial use) and after six months storage under accelerated conditions (at 40°C/75 % rH in primary packaging intended for the commercial use), in each case according to ICH guideline Q1A(R2).

In particular, ICH stability storage and testing was performed according to the applicable pharmaceutical regulatory standards as described in ICH guideline Q1A(R2), wherein the comparative and inventive pharmaceutical compositions have been packed in PVC-Alu blisters and HDPE bottles (bottle volume: 60 cm³, 56 tablets per bottle, comprising 2 g silica gel as desiccant) as primary packaging.

A 2 to 4-week Accelerated Stability Assessment Program (ASAP), according to Waterman 2011 (Waterman KC, The application of the Accelerated Stability Assessment Program (ASAP) to quality by design (QbD) for drug product stability, AAPS PharmSciTech, Vol. 12, No. 3, September 2011) is additionally also applied for a much faster estimation of chemical stability of a pharmaceutical composition compared to ICH stability program and for a precise relative comparison of chemical stability of several different pharmaceutical compositions, respectively. Said program is done at 60 °C open exposure at a relative humidity of 30 to 75 % rH, for example at a relative humidity of 40% rH.

The levels of N-Nitroso-Varenicline impurity are analyzed at initial stage and after above-mentioned ICH and ASAP storage and used for the relative comparison of chemical stability of the comparative and inventive pharmaceutical compositions (cf. following table for ASAP data).

### Analytical method:

The resulting compositions (tablets) were analyzed with respect to their content of N-nitroso-varenicline impurity at initial stage and after storage using a liquid chromategraphy with mass spectrophotometric system with gradient elution capability, mass detector and an auto sampler (Sciex QTRAP 4500 LC- MS/MS with UHPLC Exion LC separations module). As analytical column, a stainless steel column 150 mm long, 3.0 mm internal diameter filled with biphenyl chemically bonded with core shell silica particles of 2.6 µm diameter (Use Kinetex Biphenyl, 150 mm x 3.0 mm, 2.6 µ, 100 A°) is used.

Preparation of mobile phase:
Mobile phase-A: Prepare 0.1% Formic acid buffer in water. For example, transfer accurately 1.0 mL of formic acid into 1000 mL of water and mix. Filter through 0.22 µm membrane filter and degas.
Mobile phase-B: Methanol with 0.1% formic acid. For example, add 1.0 mL of formic acid into 1000 mL of methanol and mix. Filter through 0.22 µm membrane filter and degas.Diluent: Methanol.

Chromatographic conditions:
HPLC Column: Kinetex Biphenyl, 150 mm x 3.0 mm, 2.6 µ, 100 A°
Flow rate: 0.5 mL/minute
Injection volume: 3 µL
Column oven temperature: 30 °C
Sample temperature: 5°C
Data acquisition time: 15 minutes
Pumpmode: Gradient
Rinsing solution Gradient programme: Methanol: Water (80:20 v/v)

**Table 8: Gradient programme.**

| | | | | | | | | |
|---|---|---|---|---|---|---|---|---|
| Time (minutes) | 0 | 1.0 | 6.0 | 9.5 | 10.0 | 11.0 | 11.1 | 15.0 |
| Mobile phase-A (%) | 70 | 70 | 20 | 20 | 0 | 0 | 70 | 70 |
| Mobile phase-B (%) | 30 | 30 | 80 | 80 | 100 | 100 | 30 | 30 |

Mass Spectrometer Conditions:
Source type: ESI (Electrospray ionization)
Curtain gas (CUR): 30
Collision gas (CAD): Medium
Ion spray voltage (IS): 5000
Temperature (TEM): 500
Ion source gas1 (GS1): 50
Ion source gas2 (GS2): 55
Declustering potential (DP): 70
Entrance potential (EP): 10
Collison energy (CE): 22
Collison cell exit potential (CXP): 14
Scan type: MRM
Qi/Q3-Mass: 241.1/211.3
Polarity: Positive
Dwell time: 200 msec
Duration: 15 minutes
Integrated valve method properties
Valve type: Diverter

**Table 9: Mass spectrometer conditions.**

| | | |
|---|---|---|
| Total time (minutes) | 6.5 | 10.0 |
| Position | A | B |

### Preparation of solutions:

Blank solution: Use diluent as blank.

Standard Solution: Prepare a solution containing about 9.25 ng/mL of IM2 impurity (Varenicline nitroso impurity) standard in diluent (i.e., 18.5 ppm w.r.t varenicline in sample). For example, weigh about 2.31 mg of IM2 impurity standard into a 25 mL clean, dry volumetric flask. Add 15 mL of diluent and sonicate to dissolve. Make up to volume with diluent and mix. Transfer 1 mL into a 100 mL volumetric flask and make up to volume with diluent and mix. Dilute 5 mL to 50 mL with diluenl aml mix. Further, dilute 1 mL to 10 mL with diluent andmix.

QC Standard: Prepare a solution containing about 9.25 ng/mL of IM2 impurity (Varenicline nitroso impurity) standard in diluent (i.e., 18.5 ppm w.r.t varenicline in sample). For example, weigh about 2.31 mg ofIM2 impurity standard into a 25 mL clean, dry volumetric flask. Add 15 mL of diluent and sonicate to dissolve. Make up to volume with diluent and mix. Transfer 1 mL into a 100 mL volumetric flask and make up to volume with diluent and mix. Dilute 5 mL to 50 mL with diluent and mix. Further, dilute 1 mL to 10 mL with diluent and mix.

Sample solution:
For 0.5 mg strength: Weigh and transfer 20 tablets into mortar and grind to fine powder. Weigh and transfer powdered sample equivalent to 5 mg of Varenicline (i.e., equivalent to weight of 10 tablets) into clean, dry centrifuge tube or 25 mL volumetric flask. Add accurately measured 10 mL of diluent. Mix the sample for about 5 minutes on Vortex mixer and shake for 40 minutes. After extrnction, centrifuge the sample at 4500 rpm for 15 minutes. Filter through 0.22 µm PVDF or Nylon syringe filter by discarding first 4 mL of filtrate.

For 1 mg strength: Weigh and transfer 20 tablets into mortar and grind to fine powder. Weigh and transfer powdered sample equivalent to 5 mg of Varenicline (i.e., equivalent to weight of 5 tablets) into clean, dry centrifuge tube or 25 mL volumetric flask. Add accurately measured 10 mL of diluent. Mix the sample for about 5 minutes on Vortex mixer and shake for 40 minutes. After extraction, centrifuge the sample at 4500 rpm for 15 minutes. Filter through 0.22 µm PVDF or Nylon syringe filter by discarding first 4 mL of filtrate.

### Analytical results:

Table 10 shows the ASAP data of the comparative and inventive pharmaceutical compositions. According to the present experimental set up, the storage was conducted at 60 °C and 30-75 % relative humidity, e.g. 40 % relative humidity, under open exposure for 2 weeks and 4 weeks, respectively.

**Table 10: ASAP data (Open Exposure): N-Nitroso-Varenicline (IM2 impurity) levels in Varenicline Film-Coated Tablets. Impurity ppm values calculated w.r.to Varenicline free base. Limit of Detection (LOD) in finished product is 0.2 ppm, limit of quantification (LOQ) in finished product is 1.0 ppm.**

| **Example** | **Antioxidant** | **Antioxidant amount (wt.-%)** | **N-Nitroso-Varenicline in ppm** | | |
|---|---|---|---|---|---|
| **Number** | | | **Initial** | **60°C, open exposure** | |
| | | | | **2 weeks** | **4 weeks** |
| CI (Comp. Ex., 1 mg) | - | - | 44.2 | 181.9 | |
| C2 (Comp. Ex., 0.5 mg) | - | - | 50.3 | 205.2 | |
| E1 | **Ascorbic acid** | 0.1 | 21.4 | 47.3 | |
| E2 | | 0.5 | 22.6 | 40.8 | |
| E3 | | 1 | 15.4 | 29.5 | |
| E4 | | 2 | 18.7 | 25.9 | 32.1 |
| E5 | | 5 | 3.8 | 11.4 | 18.8 |
| E6 | | 10 | 1.9 | 6.2 | 9.8 |
| E7 | | 15 | 1.3 | 2.9 | 5.1 |
| E13 | **Sodium ascorbate** | 5 | 10.8 | 27.6 | |
| E14 | **Sodium metabisulfite** | 5 | 8.2 | 6.9 | |
| E9 | **Propyl gallate** | 0.25 | 8.6 | 30.5 | |
| E10 | | 0.5 | 8.4 | 27.3 | |
| Ell | | 0.75 | 10.6 | 25.8 | |
| E8 | | 1 | 9.6 | 22.7 | |
| E12 | | 1.75 | 10.4 | 24.9 | |
| E15 | | 1 | 4.8 | 16.6 | 25.1 |
| E16 | | 1 ' | 2.0 | 13.4 | 21.9 |
| E17 | | 1 | 1.6 | 9.9 | 15.2 |
| E18 | | 1 | < LOQ | 1.7 | 2.5 |
| E19 | | 1 | < LOQ | 2.3 | 3.2 |
| E20 | | 1 | 1.5 | 9.3 | 13.1 |
| E21 | | 1 | 1.1 | 6.7 | 9.3 |

The amount of N-Nitroso-Varenicline impurity in the used API batch of varenicline citrate and varenicline tartrate, respectively, was below detection limit (LOD of method for API is 0.2 ppm). The amount of N-Nitroso-Varenicline impurity measured for the comparative pharmaceutical compositions C1 and C2 of different varenicline strength initially after production was already at 44.2 ppm and 50.3 ppm, respectively, and thus in the range of the level of 50 ppm, showing the significant IM2 formation already during tablet manufacturing. The amount of N-Nitroso-Varenicline impurity measured for the comparative pharmaceutical compositions C1 and C2 after storage for 2 weeks (ASAP data) was 181.9 ppm and 205.2 ppm, respectively, showing that the comparative compositions have an unsuitable degradation profile with levels of N-Nitroso-Varenicline impurity clearly above the 50 ppm limit.

The amount of N-Nitroso-Varenicline impurity measured for the inventive pharmaceutical compositions initially after production was in each case below 50 ppm. Also, the amount of N-Nitroso-Varenicline impurity measured for the inventive pharmaceutical compositions after 2 and 4 weeks storage (ASAP data), respectively, showed that the inventive tablet compositions have a suitable low degradation profile with levels of N-Nitroso-Varenicline impurity remaining safely below 50 ppm.

The results to compositions E1 to E7 comprising ascorbic acid showed reduction of IM2 formation during production and stability with increasing content of ascorbic acid. Further, as Examples E8 to E12 show, already little amounts of propyl gallate lead to clearly reduced IM2 impurity formation during production and stability. Thus, according to the present invention, the use of propyl gallate as antioxidant is particularly preferred. The results to compositions E8 to E12 comprising propyl gallate also showed that until 1 wt.-% propyl gallate, the formation of IM2 impurity during stability decreases, wherein it surprisingly increases above 1 wt.-% propyl gallate. This shows than when using propyl gallate as antioxidant, about 1 wt.-% propyl gallate seems to be particularly preferred.

Comparison of Example 15 with Example 8 shows that pre-treatment of excipients with an antioxidant such as propyl gallate by means of a wet granulation step can further inhibit IM2 formation during production and storage of the tablets. As one can see from Examples 16 to 21, appropriate adaption of the solvent in the wet granulation step and use of an organic acid such as citric acid or tartaric acid, respectively, may even further inhibit IM2 formation. In particular, all compositions E15 to E21 prepared by using such a wet granulation step have an IM2 impurity level after 2 weeks of ASAP stress test below 18.5 ppm being the limit for this specific impurity as calculated on the basis of the health authorities' provision.

Table 11 shows the IM2 data of the comparative and inventive pharmaceutical compositions after six months storage under long term conditions (25°C/60 % rH in primary packaging) and after six months storage under accelerated conditions (at 40°C/75 % rH in primary packaging), in each case according to ICH guideline Q1A(R2) and using HDPE bottles including 2 g silica gel as well as PVC-Alu blisters as primary packaging material.

**Table 11: IM2 data of the comparative and inventive pharmaceutical compositions after 6M storage at 25°C/60 % rH and at 40°C/75% rH in both HDPE bottles and PVC/Alu blisters. Impurity ppm values calculated w.r.to Varenicline free base. Limit of Detection (LOD) in finished product is 0.2 ppm, limit of quantification (LOQ) in finished product is 1.0 ppm.**

| **Example Number** | **N-Nitroso-Varenicline in ppm** | | | | |
|---|---|---|---|---|---|
| | Initial | HDPE (25°C/60% RH-6M) | HDPE (40°C/75% RH-6M) | PVC/Alu (25°C/60% RH-6M) | PVC/Alu (40°C/75% RH-6M) |
| C1 (Comp. Ex., 1 mg) | 44.2 | 300.2 | 578.7 | 345.0 | 372.9 |
| C2 (Comp. Ex.,0.5 mg) | 50.3 | 520.0 | 1228.3 | 832.7 | 1067.7 |
| E1 | 21.4 | 306.3 | 405.2 | 308.7 | 420.6 |
| E2 | 22.6 | 229.0 | 296.9 | 208.7 | 231.2 |
| E3 | 15.4 | 128.7 | 159.4 | 116.9 | 94.6 |
| E4 | 18.7 | 81.4 | 107.3 | 77.2 | 58.0 |
| E5 | 3.8 | 6.3 | 7.9 | 3.2 | 3.9 |
| E6 | 1.9 | 3.4 | 4.0 | 1.6 | 1.8 |
| E7 | 1.3 | 1.6 | 1.7 | 1.5 | < LOQ |
| E13 | 10.8 | 16.3 | 18.7 | 15.6 | 10.6 |
| E14 | 8.2 | < LOQ | < LOQ | < LOQ | < LOQ |
| E9 | 8.6 | 48.1 | 54.0 | 48.8 | 51.6 |
| E10 | 8.4 | 40.0 | 48.6 | 41.0 | 48.1 |
| E11 | 10.6 | 35.0 | 41.6 | 35.5 | 42.5 |
| E8 | 9.6 | 18.5 | 20.5 | 16.2 | 18.9 |
| E12 | 10.4 | 34.8 | 42.2 | 31.5 | 34.2 |
| E15 | 4.8 | 3.6 | 4.6 | 6.6 | 9.2 |
| E16 | 1.6 | 1.6 | 1.9 | 3.1 | 4.6 |
| E17 | 1.3 | 1.4 | 1.6 | 2.5 | 2.8 |
| E18 | < LOQ | < LOQ | < LOQ (also: < LOQ after 1M, and after 2M) | < LOQ | 1.2 (also: < LOQ after 1M, and after 2M) |
| E19 | < LOQ | < LOQ | 1.0 (also: < LOQ after 1M, and after 2M) | 2.9 | 4.1 (also: < LOQ after 1M, 1.8 after 2M) |
| E20 | 1.5 | 3.3 | 4.3 (2.6 after 1M, 2.2 after 2M) | 4.8 | 5.7 (also: 3.3 after 1M, 2.3 after 2M) |
| E21 | 1.1 | 3.0 | 4.1 (also: 2.3 1M, 2.0 after 2M) | 3.1 | 4.4 (also: 2.6 after 1M, 2.1 after 2M) |

The amount of N-Nitroso-Varenicline impurity measured for the comparative pharmaceutical compositions C1 and C2 after six months storage at long term and accelerated conditions, respectively, is clearly above that 50 ppm limit in both HDPE bottles as well as PVC/Alu blisters, showing that the comparative compositions also have an unsuitable degradation profile under long term and accelerated conditions.

The results to compositions E1 to E7 comprising ascorbic acid showed reduction of IM2 formation during long term and accelerated stability with increasing content of ascorbic acid. Further, as Examples E8 to E12 show, already 0.5 wt.-% of the preferred antioxidant propyl gallate lead to IM2 impurity values below the limit of 50 ppm under the respective storage conditions. With regard to compositions E8 to E12, as already seen in the ASAP-test, also under six months long term and accelerated conditions, until 1 wt.-% propyl gallate, the formation of IM2 impurity during stability decreases, wherein it surprisingly increases above 1 wt.-% propyl gallate. This confirms that using about 1 wt.-% propyl gallate seems to be particularly preferred.

Further, the results to composition E14 show that sodium metabisulfite is particularly effective in reducing the formation of IM2 impurity. However, the use of sodium metabisulfite in the inventive formulation leads to the formation of other impurities and is thus less preferred.

When comparing Example 15 with Example 8, one also sees that pre-treatment of excipients with an antioxidant such as propyl gallate by means of a wet granulation step can further inhibit IM2 formation also under storage for six months at long term and accelerated conditions. For all compositions E15 to E21 produced by the use of a wet granulation step as described above, the content of IM2 purity after six months storage at long term and accelerated conditions, respectively, amounts to less the 10 ppm, both in HDPE bottles as well as PVC/Alu blisters, which is clearly below 18.5 ppm limit for this specific impurity as calculated on the basis of the health authorities' provision.

## Claims

1. A pharmaceutical composition, which is an oral solid dosage form, containing a pharmaceutical agent comprising a dialkylamino- or a trialkylamino-group and one or more pharmaceutically acceptable excipient(s),
wherein over a period of
- 2 weeks at 60 °C under open exposure at any humidity between 30 and 75 %rH, or
- 6 months at 40 °C / 75 %rH in a primary packaging, or
- 6 months at 25 °C / 60 %rH in a primary packaging,
the concentration of the corresponding N-nitroso-derivative of the pharmaceutical agent remains below 50 ppm, preferably below 30 ppm, more preferably below 18.5 ppm, even more preferably below 10 ppm, even more preferably below 5 ppm and most preferably below 2 ppm, such as below 1 ppm (relative to the weight of the free base of the pharmaceutical agent in the pharmaceutical composition).

2. The pharmaceutical composition according to claim 1 packed with a primary packaging, wherein over a period of
- 2 weeks at 60 °C under open exposure of the pharmaceutical composition as such at any humidity between 30 and 75 %rH, or
- 6 months at 40 °C / 75 %rH in the primary packaging, or
- 6 months at 25 °C / 60 %rH in the primary packaging,
the concentration of the corresponding N-nitroso-derivative of the pharmaceutical agent remains below 50 ppm, preferably below 30 ppm, more preferably below 18.5 ppm, even more preferably below 10 ppm, even more preferably below 5 ppm and most preferably below 2 ppm, such as below 1 ppm (relative to the weight of the free base of the pharmaceutical agent in the pharmaceutical composition).

3. The pharmaceutical composition according to claim 1 or 2, wherein none of the alkyl-substituents of the dialkylamino- or the trialkylamino-group is an alkyl-group with the alpha-position being a tertiary carbon atom.

4. The pharmaceutical composition according to any of claims 1 to 3, wherein the pharmaceutical agent is varenicline or a pharmaceutically acceptable salt thereof.

5. The pharmaceutical composition according to claim 4, wherein the pharmaceutically acceptable salt of varenicline is the tartrate or the citrate.

6. The pharmaceutical composition according to any of claims 1-5, wherein the solid dosage form is a tablet, capsule, pellets or granules, preferably a tablet.

7. The pharmaceutical composition according to any of claims 1-6, wherein the pharmaceutical composition comprises an antioxidant; preferably the pharmaceutical composition comprises an antioxidant in a concentration of at least 0.1 w.-%, preferably at least 0.25 w.-% (relative to the weight of the entire pharmaceutical composition).

8. The pharmaceutical composition according to claim 7, wherein the antioxidant is alpha-tocopherol, ascorbic palmitate, butylated hydroxyanisol, butylated hydroxytoluene, erythorbic acid, Vitamin E polyethylene glycol succinate, methionine, potassium metabisulfite, sodium metabisulfite, sodium ascorbate, sodium bisulfite, sodium formaldehyde sulfoxylate, or sodium sulfite, sodium thiosulfate, ascorbic acid or a salt thereof, or propyl gallate or a mixture thereof, and wherein propyl gallate is particularly preferred.

9. The pharmaceutical composition according to claim 8, wherein the antioxidant is propyl gallate in a concentration of 0.25-1.75 w.-%, preferably 0.5-1.5 w.-%, and most preferably 0.75-1.25 w.-% (relative to the weight of the entire pharmaceutical composition).

10. The pharmaceutical composition according to any of claims 1-9, wherein the composition further comprises an organic acid such as citric acid or tartaric acid.

11. The pharmaceutical composition according to any of claims 1-10, wherein the primary packaging is a pharmaceutically acceptable bottle, preferably a HDPE bottle optionally including any desiccant, or a pharmaceutically acceptable blister, preferably a PVC-Alu blister.

12. A process for the manufacture of a pharmaceutical composition according to any of claims 1 to 11, wherein (a) pharmaceutically acceptable excipient(s) such as a diluent and/or a binder are wet granulated with an aqueous dispersion, preferably an aqueous solution, of an antioxidant so that wet granules are formed, (b) after drying said wet granules, the resulting dried granules are blended with the pharmaceutical agent and optionally another pharmaceutically acceptable excipient such as a lubricant and/or an organic acid, and (c) preferably the resulting blend is dry granulated.

13. The process according to claim 12, wherein the preferably dry granulated blend (d) subsequently is either
(i) compressed to a tablet, typically together with a non-granular pharmaceutically acceptable excipient such as a lubricant, or
(ii) filled into capsules.

14. The process according to claim 12 or 13, wherein the aqueous dispersion also comprises a pharmaceutically acceptable alcohol such as ethanol.

15. The process according to any of claims 12 to 14, wherein the aqueous dispersion also comprises an organic acid such as citric acid or tartaric acid.

16. The pharmaceutical composition according to any of claims 1 to 11, wherein the composition is obtained by (a) wet granulating any pharmaceutically acceptable excipient(s) such as a diluent and/or a binder with an aqueous dispersion, preferably an aqueous solution, of an antioxidant so that wet granules are formed, (b) after drying said wet granules, the resulting dried granules are blended with the pharmaceutical agent and optionally another pharmaceutically acceptable excipient such as a lubricant and/or an organic acid, and (c) typically dry granulating the resulting blend.

17. The pharmaceutical composition according to claim 16, wherein (d) subsequently
(i) a tablet is obtained by compressing the preferably dry granulated blend, typically together with a non-granular pharmaceutically acceptable excipient such as a lubricant, or
(ii) capsules are obtained by filling the corresponding capsule shells with said preferably dry granulated blend.
